(19) **Europäisches Patentamt**

**European Patent Office**

**Office européen des brevets**

(11) **EP 0 569 616 B1**

(12) **EUROPÄISCHE PATENTSCHRIFT**

(45) Veröffentlichungstag und Bekanntmachung des Hinweises auf die Patenterteilung:
**21.04.1999 Patentblatt 1999/16**

(51) Int. Cl.$^6$: **A61N 1/39**

(21) Anmeldenummer: **92110293.5**

(22) Anmeldetag: **17.06.1992**

(54) **Implantat zur elektrischen Impulsbeaufschlagung von lebendem Gewebe**

Implant for applying electrical pulse to living tissues

Implant pour la délivrance de impulsions électriques à un tissu vivant

(84) Benannte Vertragsstaaten:
**DE FR GB IT NL SE**

(30) Priorität: **12.05.1992 EP 92107996**

(43) Veröffentlichungstag der Anmeldung:
**18.11.1993 Patentblatt 1993/46**

(73) Patentinhaber: **Pacesetter AB**
**171 95 Solna (SE)**

(72) Erfinder:
- **Hirschberg, Jakub**
  **183 44 Täby (SE)**
- **Strandberg, Hans**
  **172 36 Sundbyberg (SE)**
- **Stroetmann, Brigitte**
  **8525 Uttenreuth (DE)**
- **Lenz, Michael**
  **8011 Zorneding (DE)**

(74) Vertreter:
**Lettström, Richard Wilhelm**
**Albihns Patentbyra Stockholm AB**
**P.O. Box 5581**
**114 85 Stockholm (SE)**

(56) Entgegenhaltungen:
**EP-A- 0 026 324**     **EP-A- 0 315 368**
**DE-A- 3 715 822**     **DE-A- 3 734 036**
**DE-A- 3 910 741**     **US-A- 3 886 950**
**US-A- 5 107 834**

## Beschreibung

Die Erfindung betrifft ein Implantat zur elektrischen Impulsbeaufschlagung von lebendem Gewebe mit einer Ladekapazität, die an ihren beiden Seiten zum Aufladen an eine Ladeschaltung schaltbar ist und zum Entladen über eine steuerbare Schalteinrichtung mit mindestens zwei im Bereich des Gewebes implantierten Elektroden verbindbar ist, und mit einer Steueranordnung, die die von ihr gesteuerte Schalteinrichtung mit einer vorgegebenen Schaltfrequenz ein- und ausschaltet.

Eine derartige, aus der DE-A-37 15 822 bekannte Vorrichtung dient zum Defibrillieren oder Kardiovertieren eines Herzens. Bei der bekannten Vorrichtung wird ein Ladekondensator, der vorher durch eine Ladeschaltung auf eine vorgegebene Ladespannung aufgeladen wurde, über eine steuerbare Schalteinrichtung mit zwei im Bereich des Herzens angeordneten Elektroden verbunden. Die steuerbare Schalteinrichtung wird dabei von einer Steuereinrichtung mit einer vorgegebenen festen Frequenz im Bereich von 10 kHz bis 1 MHz ein- und ausgeschaltet, wodurch der Defibrillierungsstrom durch das Herz in eine Vielzahl von mit hoher Folgefrequenz aufeinanderfolgenden Einzelimpulsen aufgeteilt wird, deren Impulshöhe exponentiell abklingt. Durch die Beaufschlagung des Herzens mit den hochfrequenten Impulsen soll die Frequenzabhangigkeit der Impedanz des Herzgewebes zur Verteilung der Defibrillierungsenergie über das ganze Herz ausgenutzt werden, so daß eine niedrige Energie zur effektiven Defibrillation erforderlich ist.

Aus der US-A-4 834 100 ist eine weitere Vorrichtung zum Defibrillieren eines Herzens bekannt, bei der der dem Herzen zugeführte Stromimpuls den Verlauf einer stark gedämpften Sinusschwingung (sog. Lown wave) aufweist, die als besonders wirksam zur Erzielung einer Defibrillation des Herzens angesehen wird. Dieser Stromverlauf wird dadurch realisiert, daß im Entladestromkreis einer über eine steuerbare Schalteinrichtung mit Elektroden im Bereich des Herzens verbundenen Ladekapazität eine Induktivität angeordnet ist. Da die Ladekapazität einen hohen Kapazitätswert aufweist, muß die Induktivität einen entsprechend hohen Induktivitätswert aufweisen, um den gewünschten Stromverlauf zu erhalten; damit sind jedoch verhältnismäßig große Abmessungen der Induktivität verbunden, was bei implantierbaren Defibrillatoren als nachteilig anzusehen ist. Darüberhinaus ist bei dem bekannten Defibrillator der Stromverlauf durch die gewählten Kapazitäts- und Induktivitätswerte fest vorgegeben, wobei jedoch der Einfluß der Impedanz des Herzgewebes und der jeweiligen Anordnung der Elektroden auf den Stromverlauf unberücksichtigt bleibt.

Bisher sind eine Reihe von unterschiedlichen Impulsformen wie z.B. die beiden oben erwähnten Impulsformen sowie Rechteckimpulse oder exponentiell abklingende Stromimpulse in Bezug auf Ihre Effektivität zum Defibrillieren oder Kardiovertieren eines Herzens untersucht worden. Die jeweilige Form der untersuchten Stromimpulse war dabei in erster Linie durch die technischen Möglichkeiten zur Erzeugung der Impulse vorbestimmt.

Der Erfindung liegt die Aufgabe zugrunde, mit möglichst einfachen Mitteln die Erzeugung von Stromimpulsen zur Beaufschlagung von lebendem Gewebe mit einem in weiten Grenzen beliebig einstellbaren Stromverlauf zu ermöglichen, um damit einen in Bezug auf die gewünschte Wirkung der Impulsbeaufschlagung optimalen Stromverlauf einstellen zu können.

In der US-PS 3 886 950 wird ein Defibrillator beschrieben, der zur Verwendung mit an dem Körper liegenden Elektroden bestimmt ist. Die Vorrichtung weist eine Mehrzahl Von mit dem Patienten in Reihe geschalteten Kondensatoren auf. Die Vorrichtung enthält eine Steuerungsschaltung, um die Stromstärke auszugleichen, wenn mehrere Kondensatoren nacheinander entladen werden, sowie eine Steuereinrichtung, um den Gesamtstrom durch den Patienten zu begrenzen und die Kondensatoren zu entladen, die während des gegenwärtigen Verlaufes nicht entladen worden sind.

Gemäß der Erfindung wird die Aufgabe dadurch gelöst, daß in der Vorrichtung der Eingangs angegebenen Art zwischen der Schalteinrichtung und den Verbindungen zu den Elektroden Mittel zum Glätten des elektrischen Stroms durch die Elektroden angeordnet sind und daß die Steueranordnung Mittel zum Ändern der Schaltfrequenz umfaßt, die die Schaltfrequenz während der Dauer der Impulsbeaufschlagung des Gewebes in der Weise ändern, daß der die Ladekapazität entladenden Strom durch die Elektroden einen vorgegebenen, von einem exponentiell abklingenden Stromverlauf abweichenden Verlauf aufweist. Hierbei und im folgenden ist der Stromverlauf durch die Elektroden als gleichwertig mit dem Spannungsverlauf zwischen den Elektroden anzusehen. Entsprechend der variierenden Schaltfrequenz wird die Ladekapazität über die Dauer der Impulsbeaufschlagung während der unterschiedlichen Einschaltzeiten, also zeitdiskret, mit variierenden Ladungsmengen entladen, die durch die Mittel zur Stromglättung in einen Strom mit einem vorgegebenen Stromverlauf umgesetzt werden. Dabei kann innerhalb der Grenzen des exponentiell abklingenden Stromverlaufs, der sich bei einer direkten Entladung der Ladekapazität über das Gewebe ergeben würde, jeder beliebige Stromverlauf eingestellt werden.

Die Mittel zum Glätten des elektrischen Stroms können in vorteilhafter Weise aus einer zwischen den Elektroden liegenden Glättungskapazität bestehen. Dabei werden während der unterschiedlichen Einschaltzeiten der steuerbaren Schalteinrichtung unterschiedliche Ladungsmengen von der Ladekapazität auf die Glättungskapazität übertragen, die sich während der Ausschaltzeiten der Schalteinrichtung über das Gewebe zwischen den Elektroden entlädt. Je höher die variable Schaltfrequenz ist, desto kleiner kann der Kapazitätswert für die Glättungskapazität gewählt werden, um den Verlauf des elektrischen Stroms durch die Elektroden in einem vorgegebenen Maß zu glätten. Der Vorteil einer kleinen Glättungskapazität besteht außer in der geringen Baugröße auch in den geringen Ladungsverlusten beim Aufladen durch die Ladekapazität; so kann die Glättungskapazität bei 1/100stel des Kapazitätswertes der Ladekapazität auf 99% der Ladespannung der Ladekapazität aufgeladen werden.

Bei entsprechend hohen Schaltfrequenzen für die Schalteinrichtung kann in vorteilhafter Weise die Glättungskapazität von den Elektrodenleitungen gebildet werden, die die Elektroden mit der Schalteinrichtung und der Ladekapazität verbinden, so daß ein eigens zur Bildung der Glättungskapazität vorgesehenes Bauteil nicht erforderlich ist.

Alternativ zu der Glättungskapazität kann vorgesehen sein, daß die Mittel zum Glätten des elektrischen Stroms aus einer Glättungsinduktivität bestehen, die in dem Stromweg von der Ladekapazität und der Schalteinrichtung zu den Elektroden angeordnet ist, und daß ein Stromventil in Reihenschaltung mit der Glättungsinduktivität zwischen den Elektroden angeordnet ist. Das Stromventil ermöglicht einen von der Glättungsinduktivität verursachten Entmagnetisierungsstromfluß während der Ausschaltzeiten der steuerbaren Schalteinrichtung und kann aus einer Freilaufdiode oder einem steuerbaren Schalter bestehen, der gleichzeitig mit der steuerbaren Schalteinrichtung aber in entgegengesetzter Weise ein- und ausgeschaltet wird. Ebenso, wie bei der Glättungskapazität, kann der Induktivitätswert für die Glättungsinduktivität um so kleiner gewählt werden, je höher die variable Schaltfrequenz ist.

Die Mittel zum Ändern der Schaltfrequenz für die steuerbare Schalteinrichtung können im einfachsten Fall einen Impulsgeber aufweisen, der eine vorgegebene Folge von Einschalt- und Ausschaltimpulsen mit variablen Impulsdauern zum Ein- bzw. Ausschalten der steuerbaren Schalteinrichtung erzeugt. Dabei ist die Folge der Einschalt- und Ausschaltimpulse in Bezug auf einen bestimmten gewünschten Stromverlauf durch die Elektroden fest vorgegeben.

Entsprechend einer besonders vorteilhaften Weiterbildung der erfindungsgemäßen Vorrichtung ist vorgesehen, daß die Mittel zum Ändern der Schaltfrequenz eine Meßeinrichtung zum Erfassen einer dem elektrischen Strom durch die Elektroden oder der elektrischen Spannung an den Elektroden entsprechenden Meßgröße aufweisen, daß eine Vorrichtung zur Erzeugung einer dem vorgegebenen Stromverlauf entsprechenden Führungsgröße vorgesehen ist und daß eine Vergleichseinrichtung zum Vergleichen der Meßgröße mit der Führungsgröße vorgesehen ist, wobei die Vergleichseinrichtung für die steuerbare Schalteinrichtung jedesmal dann ein Einschaltsignal erzeugt, wenn die Führungsgröße die Meßgröße übersteigt, und ein Ausschaltsignal erzeugt, wenn die Führungsgröße die Meßgröße unterschreitet. Während der elektrischen Impulsbeaufschlagung des lebenden Gewebes wird also der tatsächliche Strom durch das Gewebe laufend mit dem vorgegebenen Stromverlauf verglichen und in Abhängigkeit davon die Schaltfrequenz für die steuerbare Schalteinrichtung automatisch so geregelt, daß der tatsächliche Stromverlauf an den vorgegebenen Stromverlauf angepaßt wird. Auf diese Weise wird insbesondere auch der Einfluß der jeweiligen Anordnung der Elektroden und der Herzgeometrie auf den Stromverlauf ausgeschaltet. Um die Schaltfrequenz für die steuerbare Schalteinrichtung zu begrenzen, kann für die Erzeugung des Ein- bzw. Ausschaltsignals für die steuerbare Schalteinrichtung eine Schalthysterese vorgesehen werden.

Gemäß einer vorteilhaften Weiterbildung der erfindungsgemäßen Vorrichtung ist eine Impedanzmeßeinrichtung zur Erfassung der elektrischen Impedanz zwischen den Elektroden während vorgegebener Meßzeiten und zur Steuerung der Mittel zur Änderung der Schaltfrequenz in Abhängigkeit von der gemessenen Impedanz vorgesehen. Mit der Messung der elektrischen Impedanz zwischen den Elektroden wird eine Information über die Anordnung der Elektroden und die Geometrie des zwischen den Elekroden liegenden lebenden Gewebes erhalten und in Abhängigkeit davon ein geeigneter Stromverlauf für die elektrische Impulsbeaufschlagung des Gewebes ausgewählt. Dies kann in der Weise geschehen, daß in Abhängigkeit von der gemessenen Impedanz eine bestimmte Folge von Ein- und Ausschaltimpulsen für die steuerbare Schalteinrichtung zur Erzielung eines vorgegebenen Stromverlaufs direkt vorgegeben wird oder daß im Falle der oben angegebenen automatischen Regelung des Stromverlaufs die Führungsgröße in Abhängigkeit von der gemessenen Impedanz vorgegeben wird.

Alternativ zur Impedanzmessung kann auch die oben angegebene Meßeinrichtung zum Erfassen des elektrischen Stroms durch die Elektroden zum Beschaffen einer Information über die jeweilige Anordnung der Elektroden und die Geometrie des Gewebes zwischen den Elektroden herangezogen werden, indem nach dem Aufladen der Ladekapazität auf eine Meßspannung ein Einschaltimpuls oder eine vorgegebene Folge von Ein- und Ausschaltimpulsen für die steuerbare Schalteinrichtung erzeugt wird und der dabei erzeugte Stromfluß über die Elektroden gemessen wird.

Um bei implantierbaren Geräten, wie z.B. einem implantierbaren Defibrillator, den gewünschten Stromverlauf programmieren zu können, ist entsprechend einer vorteilhaften Weiterbildung der erfindungsgemäßen Vorrichtung vorgesehen, daß die Mittel zum Ändern der Schaltfrequenz mit einem Parameterspeicher verbunden sind, in dem Parameterwerte für die Änderung der Schaltfrequenz abgespeichert sind, und daß der Parameterspeicher mit einer Telemetrieeinrichtung zur Übertragung der Parameterwerte zwischen dieser und einem Programmiergerät verbunden ist. Für den Fall, daß die vorstehend genannte Impedanzmeßeinrichtung oder eine sonstige Meßeinrichtung zur Bestimmung der Elektrodenanordnung und der Gewebegeometrie vorgesehen ist, können die entsprechenden Informationen mittels der Telemetrieeinrichtung an das Programmiergerät übertragen werden und dort zur Anzeige gebracht werden, um der Bedienperson Hinweise zur Programmierung der Parameterwerte für einen bestimmten Stromverlauf zu geben.

Aus verschiedenen Gründen können die Elektroden oft nicht an den für sie optimalen Stellen in bezug auf das mit dem elektrischen Impuls zu beaufschlagende Gewebe plaziert werden, um eine gleichmäßige Stromverteilung in dem Gewebe zu erreichen; hinzu kommt, daß das Gewebe in der Regel eine ungleichmäßige Massenverteilung aufweist. So ergibt sich insbesondere bei der Defibrillation oder Kardiovertierung des Herzens das Problem, daß bestimmte Bereiche des Herzens nicht oder nur unzureichend von dem Defibrillierungsstrom durchsetzt werden und daher nicht defibrilliert werden. Um eine sowohl räumlich als auch zeitlich optimale Stromverteilung in dem impulsbeaufschlagten Gewebe zu erzielen, ist im Rahmen der Erfindung vorgesehen, daß die Ladekapazität oder zumindest eine weitere, separat aufladbare Ladekapazität an ihren beiden Seiten an eine weitere steuerbare Schalteinrichtung mit einer der Elektroden und einer weiteren Elektrode verbunden ist, daß zwischen der weiteren Schalteinrichtung und der Elektrode und der weiteren Elektrode Mittel zum Glätten des elektrischen Stroms durch die weitere Elektrode angeordnet sind und daß eine weitere Steueranordnung zum Ein- und Ausschalten der weiteren steuerbaren Schalteinrichtung mit einer änderbaren Schaltfrequenz vorgesehen ist. Dadurch ist es möglich, daß Gewebe über die zumindest drei Elektroden mit unterschiedlichen, gleichzeitigen oder einander zeitlich überlappenden Stromverläufen zu beaufschlagen, so daß eine Stromverteilung erreicht wird, die je nach Einstellung der gewünschten Stromverläufe zu unterschiedlichen Zeitpunkten unterschiedliche Bereiche des Gewebes in unterschiedlicher Richtung und mit unterschiedlicher Stromdichte durchsetzt. Insbesondere bei dieser Weiterbildung der erfindungsgemäßen Vorrichtung erweist sich die vorstehend angegebene Meßeinrichtung zur Bestimmung der Anordnung der Elektroden und der Gewebegeometrie als besonders vorteilhaft, weil die so erhaltenen Informationen zur gezielten Einstellung der Stromverteilung über dem Gewebe herangezogen werden können.

Zur weiteren Erläuterung der Erfindung wird im folgenden auf die Figuren der Zeichnung Bezug genommen. Im einzelnen zeigen

FIG. 1    ein erstes Ausführungsbeispiel der erfindungsgemäßen Vorrichtung,

FIG. 2    ein Beispiel für den mit der Vorrichtung nach FIG 1 einstellbaren Stromverlauf,

FIG. 3    ein zweites Beispiel der erfindungsgemäßen Vorrichtung,

FIG. 4    ein Beispiel für den mit der Vorrichtung nach FIG. 3 einstellbaren Stromverlauf,

FIG. 5    ein drittes Ausführungsbeispiel der erfindungsgemäßen Vorrichtung,

FIG. 6    ein viertes Ausführungsbeispiel der erfindungsgemäßen Vorrichtung,

FIG. 7    Beispiele für die mit der Vorrichtung nach FIG. 6 einstellbaren Spannungs- und Stromverläufe,

FIG. 8    ein fünftes Ausführungsbeispiel der erfindungsgemäßen Vorrichtung,

FIG. 9    ein Beispiel für die mit der Vorrichtung nach FIG. 8 einstellbaren Stromverläufe und

FIG. 10   ein Schaltungsbeispiel zur Erzeugung biphasischer Impulse.

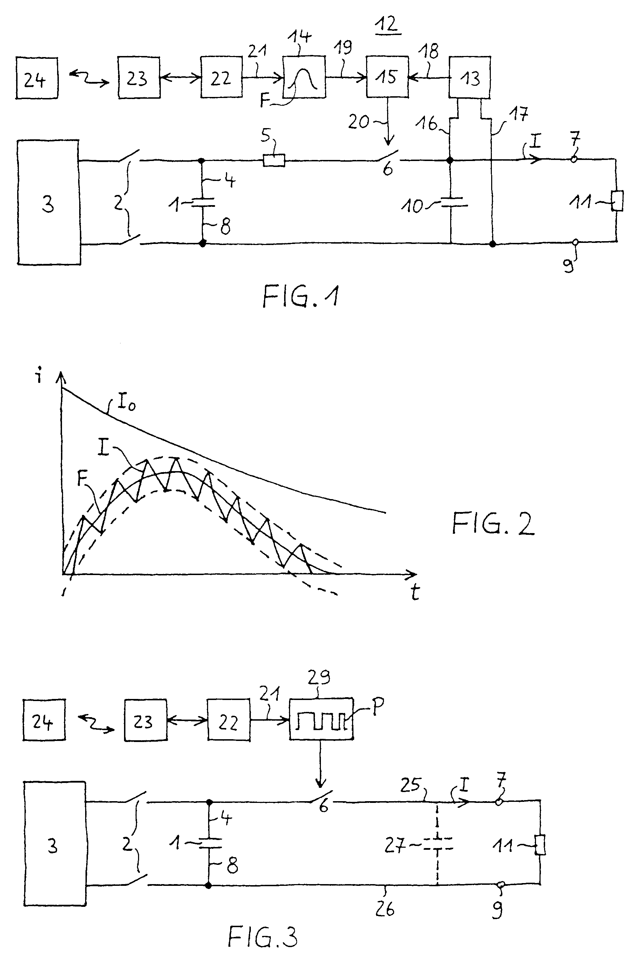

Figur 1 zeigt ein erstes Beispiel des erfindungsgemäßen Implantats, wobei es sich vorzugsweise um einen implantierbaren Defibrillator oder Kardiovertierer handelt. Eine Ladekapazität 1 ist zum Aufladen auf eine vorgegebene Ladespannung über eine steuerbare Schalteranordnung 2 an eine Ladeschaltung 3 schaltbar. Die Ladekapazität 1 ist auf einer Seite 4 über einen strombegrenzenden Widerstand 5 und eine steuerbare Schalteinrichtung 6 mit einer Elektrode 7 verbunden und auf der anderen Seite 8 direkt an einer zweiten Elektrode 9 angeschlossen. Zwischen den beiden Elektroden 7 und 9 ist eine Glättungskapazität 10 angeordnet. Die beiden Elektroden 7 und 9 sind an einem mit einem elektrischen Impuls zu beaufschlagenden lebenden Gewebe angeordnet, daß hier durch seine elektrische Impedanz 11 zwischen den Elektroden 7 und 9 verdeutlicht ist. Die steuerbare Schalteinrichtung 6 wird von einer Steueranordnung 12 angesteuert, die aus einer Spannungsmeßeinrichtung 13, einer Vorrichtung 14 zur Erzeugung einer Führungsgröße F und einer Vergleichseinrichtung 15 besteht. Die Spannungsmeßeinrichtung 13 ist über zwei Eingangsleitungen 16 und 17 mit den beiden Elektroden 7 und 9 verbunden und ausgangsseitig an einem ersten Eingang 18 der Vergleichseinrichtung 15 angeschlossen. Die Vorrichtung 14 zur Erzeugung der Führungsgröße F ist an einem zweiten Eingang 19 der Vergleichseinrichtung 15 angeschossen, die über eine Ausgangssteuerleitung 20 die steuerbare Schalteinheit 6 ansteuert. Die Vorrichtung 14 zur Erzeugung der Führungsgröße F ist über eine Steuerleitung 21 mit einem Parameterspeicher 22 verbunden, in dem Parameterwerte zur Erzeugung der Führungsgröße F abgespeichert sind. Diese Parameterwerte können mittels einer an dem Parameterspeicher 22 angeschlossenen Telemetrieeinrichtung 23 zwischen dieser und einem Programmiergerät 24 übertragen werden.

Zur Impulsbeaufschlagung des Gewebes 11 wird zunächst über eine nicht gezeigte übergeordnete Steueranordnung die steuerbare Schalteranordnung 2 geschlossen und die Ladekapazität 1 von der Ladeschaltung 3 auf eine vorgegebene Ladespannung aufgeladen. In dem Parameterspeicher 2 sind Parameterwerte enthalten, die dem gewünschten zu erzeugenden Stromverlauf I im Gewebe 11 entsprechen und mittels des Programmiergeräts 24 und der Telemetrieeinrichtung 23 in den Parameterspeicher 22 eingelesen worden sind. Die an dem Parameterspeicher 22 angeschlossene Vorrichtung 14 erzeugt während der Impulsbeaufschlagung des Gewebes 11 aus den Parameterwerten die Führungsgröße F, deren Verlauf dem gewünschten Stromverlauf I entspricht. Gleichzeitig wird mit der Spannungsmeßeinrichtung 13 die elektrische Spannung über dem Gewebe 11 und damit weitestgehend auch der Strom I durch das Gewebe 11 erfaßt und die so ermittelte Meßgröße dem ersten Eingang 18 der Vergleichseinrichtung 15 zugeführt. Die Vergleichseinrichtung 15 vergleicht die Meßgröße mit der Führungsgröße F an ihrem zweiten Eingang 19 und erzeugt über ihre Ausgangssteuerleitung 20 jedemal dann ein Einschaltsignal für die steuerbare Schalteinrichtung 6, wenn die Führungsgröße F die Meßgröße um einen vorgegebenen ersten Betrag übersteigt. Unterschreitet die Führungsgröße F die Meßgröße um einen zweiten vorgegebenen Betrag, so erzeugt die Vergleichseinrichtung 15 ein Ausschaltsignal für die steuerbare Schalteinrichtung 6. Der daraus resultierende Stromverlauf I über die Elektroden 7 und 9 ist in Figur 2 dargestellt.

In dem Diagramm nach Figur 2 ist mit F die von der Vorrichtung 14 erzeugte und dem gewünschten Stromverlauf entsprechende Führungsgröße über der Zeit t bezeichnet und mit $I_0$ der exponentiell abklingende Stromverlauf bezeichnet, der sich ergeben würde, wenn die auf die Ladespannung aufgeladene Ladekapazität 1 nach bekannter Weise direkt über das Gewebe 11 entladen würde. Die im Abstand des ersten Betrages und des zweiten Betrages beidseitig der Führungsgröße F verlaufenden gestrichelten Kurven bezeichnen die Schalthysterese, innerhalb derer der tatsächliche Stromverlauf I über die Elektroden 7 und 9 durch die in Figur 1 gezeigte Vorrichtung an den der Führungsgröße F entsprechenden gewünschten Stromverlauf angepaßt wird. Zu Beginn der Impulserzeugung ist die steuerbare Schalteinrichtung 6 noch geöffnet und die Meßgröße gleich Null, während die Führungsgröße F entsprechend ihrem vorgegebenen Verlauf ansteigt. Sobald die Führungsgröße F den Wert der Meßgröße um den vorgegebenen ersten Betrag übersteigt, wird die steuerbare Schalteinrichtung 6 geschlossen, wobei durch die Ladekapazität 1 gleichzeitig ein Strom I durch das Gewebe 11 erzeugt wird und die Glättungskapazität 10 aufgeladen wird. Die Spannung an der Glättungskapazität 10, deren Kapazitätswert größenordnungsmäßig etwa 1/100stel des Wertes für die Ladekapazität 1 entspricht, steigt sehr schnell an und kann für den Fall, daß die steuerbare Schalteinrichtung 6 geschlossen bleibt bis auf 99% der ursprünglichen Ladespannung an dem Ladekondensator 1 ansteigen. Sobald jedoch der Strom I durch die Elektroden 7 und 9 die Führungsgröße F um den zweiten Betrag übersteigt, wird die steuerbare Schalteinrichtung 6 geöffnet, und die Aufladung des Glättungskondensators 10 durch die Ladekapazität 1 unterbrochen. Die Ladekapazität 10 entlädt sich nunmehr über das Gewebe 1, wobei die Spannung an der Glättungskapazität 10 bzw. der Strom I durch das Gewebe 11 mit einer durch die Glättungskapazität 10 und die Gewebeimpedanz 11 vorgegebenen Zeitkonstante abklingt. Sobald dabei der Strom I die Führungsgröße F wieder um den ersten Betrag untersteigt, wird die steuerbare Schalteinrichtung 6 erneut geschlossen und die Glättungskapazität 10 wieder aufgeladen. Dieser Vorgang wiederholt sich während der gesamten Dauer der Impulsbeaufschlagung des Gewebes 11, wobei der Strom I durch das Gewebe 11 innerhalb der durch die Schalthysterese vorgegebenen Grenzen dem Verlauf der Führungsgröße F folgt. Wie Figur 2 zeigt, weist der bei der herkömmlichen Entladung der Ladekapazität erzeugte Stromverlauf $I_0$ zu Beginn der Entladung seinen höchsten Wert auf, um erst nach einiger Zeit unter die zur Reizung oder Defibrillation des Gewebes erforderliche Mindeststromstärke abzufallen. Während der Anfangswert des Stromverlauf $I_0$ erheblich über dieser Mindeststromstärke liegen kann und bei einer Defibrillation des Gewebes zu Schädigungen des Gewebes führen kann, ist es mit der erfindungsgemäßen Vorrichtung möglich, den Stromverlauf I auf für das Gewebe unschädliche, aber zur Stimulation oder Defibrillation ausreichende Werte zu begrenzen.

Figur 3 zeigt eine gegenüber dem Ausführungsbeispiel nach Figur 1 vereinfachte Ausführungsform der erfindungsgemäßen Vorrichtung. Dabei sind im Vergleich zu Figur 1 gleiche Teile der Vorrichtung mit gleichen Bezugszeichen versehen. Eine Ladekapazität 1 ist über eine steuerbare Schalteranordnung 2 mit einer Ladeschaltung 3 zum Aufladen der Ladekapazität 1 auf eine vorgegebene Ladespannung verbunden. Die Ladekapazität 1 ist auf einer Seite 4 über eine steuerbare Schalteinrichtung 6 und eine erste Elektrodenleitung 25 mit einer ersten Elektrode 7 verbunden und auf der zweiten Seite 8 über eine zweite Elektrodenleitung 26 an einer zweiten Elektrode 9 angeschlossen. Anstelle des in Figur 1 gezeigten diskreten Elemente für die Glättungskapazität 10 bilden bei dem Ausführungsbeispiel nach Figur 3 die beiden Elektrodenleitungen 25 und 26 mit den Elektroden 7 und 9 die Glättungskapazität 27. Zwischen den beiden Elektroden 7 und 9 liegt das hier durch seine elektrische Impedanz 11 verdeutlichte zu stimulierende Gewebe. Zur Steuerung der steuerbaren Schalteinrichtung 6 ist diese mit der Ausgangssteuerleitung 28 eines Impulsgebers 29 verbunden, der über eine Steuerleitung 21 an einem Parameterspeicher 22 angeschlossen ist, in dem Parameterwerte für den jeweiligen Impulsbeginn und die jeweilige Impulsdauer einer Folge P von durch den Impulsgeber 29 zu erzeugenden Impulsen abgespeichert sind. Diese Parameterwerte können mittels einer an dem Parameterspeicher 22 angeschlossenen Telemetrieeinrichtung 23 zwischen den Parameterspeicher 22 und einem Programmiergerät 24 übertragen werden.

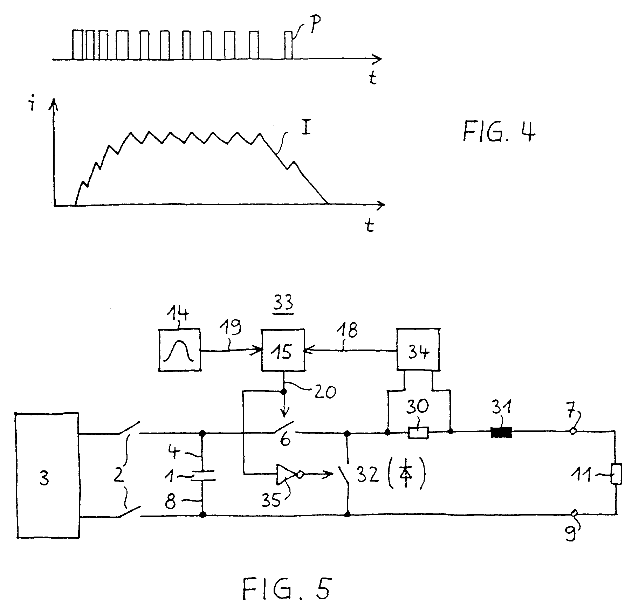

Figur 4 zeigt in einem oberen Diagramm ein Beispiel für die von dem Impulsgeber 29 zur Steuerung der steuerbaren Schalteinrichtung 6 abgegebenen Impulsfolge P und in einem unteren Diagramm den daraus resultierenden Stromverlauf I über das Gewebe 11.

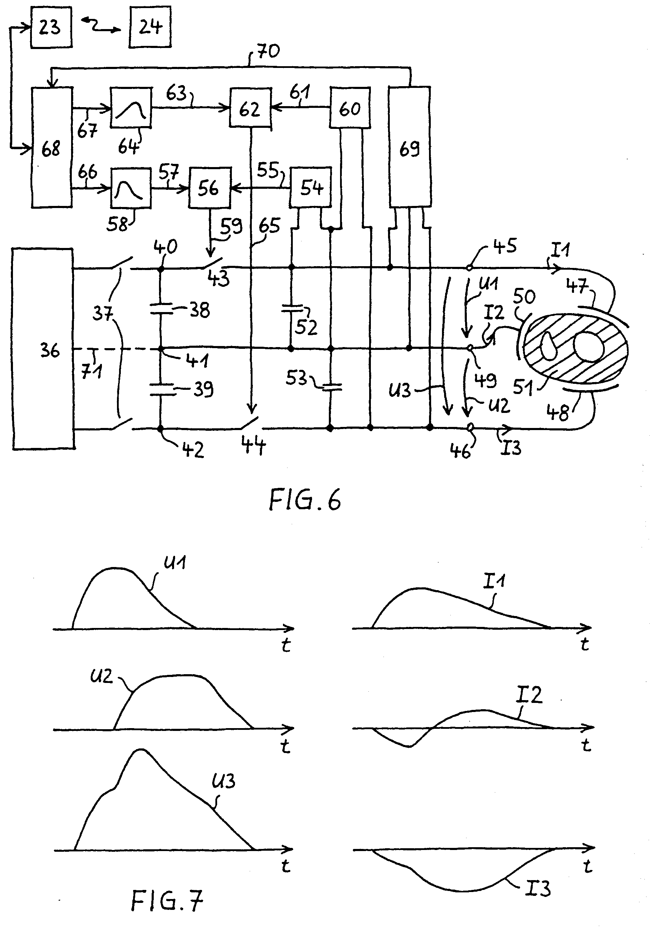

Figur 5 zeigt ein Ausführungsbeispiel für die erfindungsgemäße Vorrichtung, bei dem eine Ladekapazität 1 an ihren beiden Seiten 4 und 8 über eine steuerbare Schalteranordnung 2 an eine Ladeschaltung 3 schaltbar ist. Die Ladekapazität 1 ist an ihrer mit 4 bezeichneten Seite über eine steuerbare Schalteinrichtung 6, einen Strommeßwiderstand 30 und eine Glättungsinduktivität 31 mit einer ersten Elektrode 7 verbunden und an ihrer anderen Seite 8 direkt an einer zweiten Elektrode 9 angeschlossen. Zwischen den Elektroden 7 und 9 liegt das zu stimulierende Gewebe mit seiner Impedanz 11. Ein Stromventil 32 in Form eines steuerbaren Schalters ist derart angeordnet, daß es in Reihe mit dem Strommeßwiderstand 30 und der Glättungsinduktivität 31 zwischen den Elektroden 7 und 9 liegt. Wie in Figur 5 angedeutet ist, kann der steuerbare Schalter 32 auch durch eine Freilaufdiode ersetzt werden. Die Steuerung der steuerbaren Schalteinrichtung 6 und des steuerbaren Schalters 32 erfolgt durch eine Steueranordnung 33, die aus einer Strommeßeinrichtung, einer Vergleichseinrichtung 15 und einer Vorrichtung 14 zur Erzeugung einer Führungsgröße besteht. Die Strommeßeinrichtung besteht aus dem Meßwiderstand 30 und einer den von einem Strom durch den Meßwiderstand 30 erzeugten Spannungsabfall erfassenden Spannungsmeßeinheit 34, die ausgangsseitig mit einem ersten Eingang 18 der Vergleichseinrichtung 15 verbunden ist. Die Vorrichtung 14 zur Erzeugung einer Führungsgröße ist mit einem zweiten Eingang 19 der Vergleichseinrichtung 15 verbunden, die über ihre Ausgangssteuerleitung 20 jedesmal dann ein Einschaltsignal für die steuerbare Schalteinrichtung 6 erzeugt, wenn die Führungsgröße die von der Spannungsmeßeinheit 34 erzeugte Meßgröße um einen vorgegebenen ersten Betrag übersteigt. Unterschreitet dagegen die Führungsgröße die Meßgröße um einen zweiten vorgegebenen Betrag, so erzeugt die Vergleichseinrichtung 15 ein Ausschaltsignal für die steuerbare Schalteinrichtung 6. Der steuerbare Schalter 32 ist steuerseitig über einen Invertierer 35 mit der Ausgangssteuerleitung 20 verbunden, so daß der steuerbare Schalter 32 entgegengesetzt zur steuerbaren Schalteinrichtung 6 ein- und ausgeschaltet wird. Die Funktion der Vorrichtung nach Figur 5 ist entsprechend der nach Figur 1 mit dem Unterschied, daß die Stromglättung durch die Glättungsinduktivität 31 im Zusammenwirken mit dem Stromventil 32 erfolgt.

Das in Figur 6 gezeigte Ausführungsbeispiel der erfindungsgemäßen Vorrichtung stellt einen implantierbaren Defibrillator dar, bei dem eine Ladeschaltung 36 über eine steuerbare Schalteranordnung 37 an zwei in Reihe liegende Ladekapazitäten 38 und 39 schaltbar ist. Die Reihenschaltung der Ladekapazitäten 38 und 39 weist drei unterschiedliche Anschlußstellen 40,41 und 42 auf, wovon die beiden äußeren Anschlußstellen 40 und 42 jeweils über steuerbare Schalteinrichtungen 43 und 44 mit jeweils einem Ausgangsanschluß 45 bzw. 46 zum Anschluß von Elektroden 47 bzw. 48 verbunden sind; die mittlere Anschlußstelle 41 ist direkt mit einem Anschluß 49 für eine dritte Elektrode 50 verbunden. Die Elektroden 47,48 und 50 sind im Bereich eines hier im Querschnitt dargestellten Herzens 51 angeordnet. Zwischen den Ausgangsanschlüssen 45 und 49 sowie zwischen den Ausgangsanschlüssen 49 und 46 ist jeweils eine Glättungskapazität 52 bzw. 53 angeordnet. Eine Meßeinrichtung 54 zur Messung der elektrischen Spannung zwischen den Ausgangsanschlüssen 45 und 49 ist mit einem ersten Eingang 55 einer ersten Vergleichseinrichtung 56 verbunden, an deren zweiten Eingang 57 eine erste Vorrichtung 58 zur Erzeugung einer ersten Führungsgröße angeschlossen ist. Die Vergleichseinrichtung 56 weist eine Ausgangssteuerleitung 59 zur Steuerung der ersten Schalteinrichtung 43 auf. Eine zweite Meßeinrichtung 60 zur Messung der elektrischen Spannung zwischen den Ausgangsanschlüssen 49 und 46 ist ausgangsseitig mit einem ersten Steuereingang 61 einer zweiten Vergleichseinrichtung 62 verbunden, an deren zweiten Steuereingang 63 eine zweite Vorrichtung 64 zur Erzeugung einer zweiten Führungsgröße angeschlossen ist. Die zweite Vergleichseinrichtung 62 steuert über eine Ausgangssteuerleitung 65 die zweite steuerbare Schalteinrichtung 44. Die beiden Vorrichtungen 58 und 64 zur Erzeugung unterschiedlicher Führungsgrößen sind jeweils über Steuerleitungen 66 und 67 mit einem Parameterspeicher 68 verbunden, in dem unterschiedliche Parameterwerte für die Erzeugung der beiden unterschiedlichen Führungsgrößen gespeichert sind. Diese Parameterwerte können mittels einer an dem Parameterspeicher 68 angeschlossenen Telemetrieeinrichtung 23 zwischen dem Parameterspeicher 68 und einem externen Programmiergerät 24 übertragen werden.

An den Ausgangsanschlüssen 45,46 und 49 ist eine Impedanzmeßeinrichtung 69 angeschlossen, die zu vorgegebenen Zeitpunkten, vorzugsweise unmittelbar vor der Impulsbeaufschlagung des Herzens 51 die elektrische Impedanz des Herzgewebes 51 zwischen den Elektroden 47,48 und 50 mißt. Das in der Impedanzmeßeinrichtung 69 ausgewertete Impedanzmeßsignal wird über eine Steuerleitung 70 dem Parameterspeicher 68 zur automatischen Anpassung der Parameterwerte an die gemessene Impedanz zugeführt. Alternativ kann das Ergebnis der Impedanzmessung über die Telemetrieeinrichtung 23 zu dem Programmiergerät 24 übertragen und dort für die Bedienperson zur Anzeige gebracht werden um der Bedienperson Hinweise zur Programmierung der Parameterwerte zu geben. Auf diese Weise wird die zufällige Anordnung der Elektroden 47,48 und 50 und die Massenverteilung des Herzgewebes 51 bei der Festlegung der Parameterwerte berücksichtigt.

Durch Schließen der steuerbaren Schalteranordnung 37 werden die beiden Ladekapazitäten 38 und 39 durch die Ladeschaltung 36 in Abhängigkeit von dem Verhältnis ihrer Kapazitätswerte auf unterschiedliche Ladespannungen aufgeladen. Alternativ hierzu können die Ladekapazitäten 38 und 39 auch auf von ihrem Kapazitätsverhältnis unabhängige Ladespannungen aufgeladen werden, wozu die Ladeschaltung 36 für jede Ladekapazität 38 und 39 jeweils eine Ladespannung liefert und die beiden unterschiedlichen Ladespannungen über die um die hier gestrichelt gezeichnete Verbindung 71 erweiterte Schalteranordnung 37 an die Ladekapazität 38 und 39 geschaltet werden.

In dem Parameterspeicher 68 sind Parameterwerte enthalten, die den gewünschten zu erzeugenden Spannungsverläufen U1 und U2 zwischen den Elektroden 47 und 50 bzw. 50 und 48 entsprechen und mittels des Programmiergeräts 24 und der Telemetrieeinrichtung 23 in den Parameterspeicher 68 eingelesen worden sind. Die Spannung zwischen den Elektroden 47 und 48 ergibt sich dabei mit U3 = U1 + U2 . Die an dem Parameterspeicher 68 angeschlossenen Vorrichtungen 58 und 64 erzeugen aus den Parameterwerten Führungsgrößen, die den gewünschten Spannungsverläufen 41 und 42 entsprechen. Diese Führungsgrößeb werden in den Vergleichseinrichtungen 56 und 62 mit den von den Meßeinrichtungen 54 und 60 erfaßten tatsächlichen Spannungsverläufen U1 und U2 verglichen und zur Steuerung der steuerbaren Schalteinrichtungen 43 und 44 in der Weise herangezogen, wie es oben z.B. für die Figur 1 beschrieben ist.

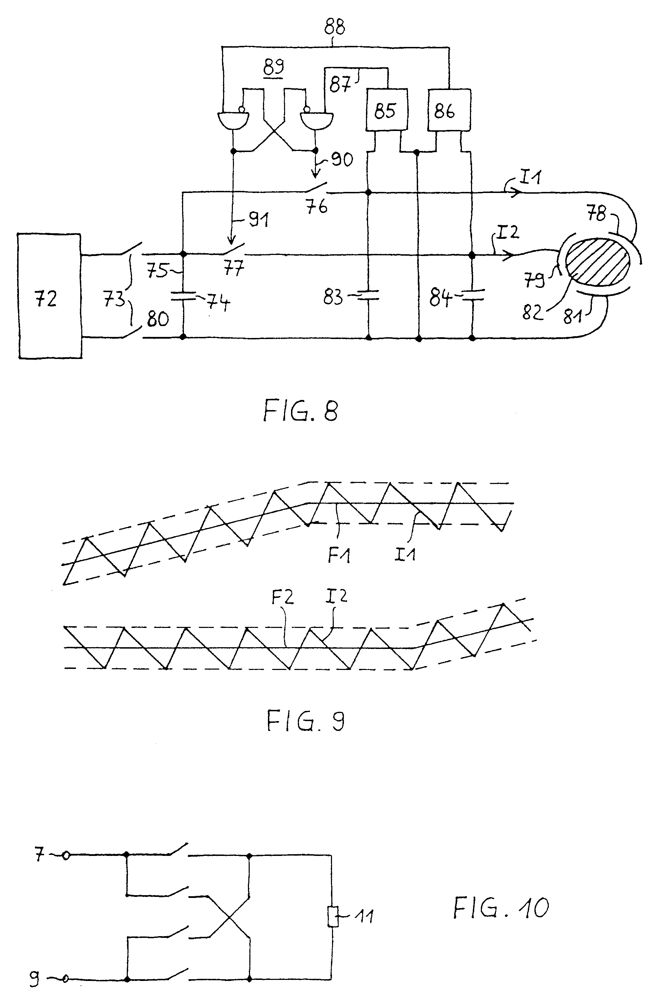

In Figur 7 ist ein Beispiel für die Spannungsverläufe U1, U2 und U3 und die daraus resultierenden Ströme I1, I2 und I3 durch die Elektroden 47, 50 und 48 dargestellt. Wie zu sehen ist, werden dabei unterschiedliche, im Falle des Stromes I2 sogar gezielt biphasische Stromverläufe erreicht werden, wobei in vorherbestimmbarer Weise unterschiediche Zeiten von unterschiedlichen Stromdichten in unterschiedliche Richtung durchsetzt werden. Dadurch können unterschiedliche Bereiche des Herzgewebes 51 nacheinander gezielt defibrilliert werden.

Bei dem in Figur 8 gezeigten Ausführungsbeispiel der erfindungsgemäßen Vorrichtung ist eine Ladeschaltung 72 über eine steuerbare Schalteranordnung 73 an eine Ladekapazität 74 schaltbar, die auf einer Seite 75 über zwei steuerbare Schalteinrichtungen 76 und 77 mit jeweils zwei Elektroden 78 und 79 und auf der anderen Seite 80 mit einer dritten Elektrode 81 verbunden ist. Die drei Elektroden sind im Bereich eines zu stimulierenden oder auf sonstige Weise mittels Stromstößen zu behandelnden lebenden Gewebes 82 angeordnet. Zwischen den mit den steuerbaren Schalteinrichtungen 76 und 77 verbundenen Elektroden 78 und 79 und der dritten Elektrode 81 sind Glättungskapazitäten 83 und 84 angeordnet. Die Steuerung der beiden Schalteinrichtungen 76 und 77 erfolgt mittels zweier Steueranordnungen 85 und 86, die bezüglich ihres Aufbaus den in Figur 6 gezeigten Steueranordnungen mit den Schaltungsblöcken 54 bis 58 und 60 bis 64 entsprechen. Die Ausgangssteuerleitungen 87 und 88 der Steueranordnungen 85 und 86 sind über eine Verriegelungsschaltung 89 mit Steuereingängen 90 und 91 der beiden steuerbaren Schalteinrichtungen 76 und 77 verbunden.

Die Funktionsweise der in Figur 8 gezeigten Vorrichtung ist im Prinzip dieselbe wie bei der nach Figur 6. Als einziger Unterschied erfolgt die Strombeaufschlagung der drei Elektroden 78,79 und 81 aus einer einzigen Energiequelle, nämlich der Ladekapazität 74, weswegen die Verriegelungsschaltung 89 vorgesehen ist, die ein gleichzeitiges Schließen der beiden steuerbaren Schalteinrichtungen 76 und 77 verhindert. Figur 9 verdeutlicht dies an Hand eines kurzen Ausschnittes aus den Stromverläufen I1 und I2, die den Führungsgrößen F1 und F2 folgen. Die gestrichelten Linien beiderseits der Führungsgrößen F1 und F2 bezeichnen die Schalthysterese der Stromregelung, die wie für Figur 1 beschrieben erfolgt.

Die erfindungsgemäße Vorrichtung dient allgemein zur elektrischen Impulsbeaufschlagung und speziell zur Defibrillation von lebendem Gewebe mit beliebig einstellbaren Impulsverläufen. Wenn biphasische Spannungsverläufe, d.h. Spannungsverläufe mit wechselnder Polarität an den Elektroden erzeugt werden sollen, so kann ohne weiteres am Ausgang der erfindungsgemäßen Vorrichtung eine Schaltung zur Spannungsumkehr beispielsweise eine Brückenschaltung mit steuerbaren Schaltern vorgesehen werden, wie dies in Figur 10 für die Vorrichtung nach Figur 1 dargestellt ist.

**Bezugszeichenliste**

| 1 | Ladekapazität |
|---|---|
| 2 | steuerbare Schalteranordnung |
| 3 | Ladeschaltung |
| 4 | eine Seite von 1 |
| 5 | Widerstand |
| 6 | steuerbare Schaltereinrichtung |
| 7 | Elektrode |
| 8 | andere Seite von 1 |
| 9 | zweite Elektrode |
| 10 | Gättungskapazität |
| 11 | lebendes Gewebe (Gewebeimpedanz) |
| 12 | Steueranordnung |

| | |
|---|---|
| 13 | Spannungsmeßeinrichtung |
| 14 | Vorrichtung zur Erzeugung einer Führungsgröße F |
| 15 | Vergleichseinrichtung |
| 16,17 | Eingangsleitungen von 13 |
| 18,19 | Eingäge von 15 |
| 20 | Ausgangssteuerleitung von 15 |
| 21 | Steuerleitung |
| 22 | Parameterspeicher |
| 23 | Telemetrieeinrichtung |
| 24 | Programmiergerät |
| 25,26 | Elektrodenleitungen |
| 27 | Glättungskapazität |
| 28 | Ausgangssteuerleitung von 29 |
| 29 | Impulsgeber |
| 30 | Stromwiderstand |
| 31 | Glättungsinduktivität |
| 32 | Stromventil (Schalter oder Diode) |
| 33 | Steueranordnung |
| 34 | Spannungsmeßeinheit |
| 35 | Invertierer |
| 36 | Ladeschaltung |
| 37 | steuerbare Schalteranordnung |
| 38,39 | Ladekapazitäten |
| 40,41,42 | Anschlußstellen von 38,39 |
| 43,44 | steuerbare Schalteinrichtungen |
| 45,46 | Ausgangsanschlüsse |
| 47,48 | Elektroden |
| 49 | Anschluß |
| 50 | dritte Elektrode |
| 51 | Herz |
| 52,53 | Glättungskapazitäten |
| 54 | Meßeinrichtung |
| 55 | erster Eingang von 56 |
| 56 | Vergleichseinrichtung |
| 57 | zweiter Eingang von 56 |
| 58 | Vorrichtung zur Erzeugung einer Führungsgröße |
| 59 | Ausgangssteuerleitung |
| 60 | Meßeinrichtung |
| 61 | erster Steuereingang von 62 |
| 62 | Vergleichseinrichtung |
| 63 | zweiter Steuereingang von 62 |
| 64 | Vorrichtung zur Erzeugung einer zweiten Führungsgröße |
| 65 | Ausgangssteuerleitung von 62 |
| 66,67 | Steuerleitungen |
| 68 | Parameterspeicher |
| 69 | Impedanzmeßeinrichtung |
| 70 | Steuerleitung |
| 71 | Verbindung |
| 72 | Ladeschaltung |
| 73 | steuerbare Schalteranordnung |
| 74 | Ladekapazität |
| 75 | eine Seite von 74 |
| 76,77 | steuerbare Schalteinrichtungen |
| 78,79 | Elektroden |
| 80 | andere Seite von 74 |
| 81 | dritte Elektrode |
| 82 | lebendes Gewebe |
| 83,84 | Glättungskapazitäten |
| 85,86 | Steueranordnungen |
| 87,88 | Ausgangssteuerleitungen von 85,86 |
| 90,91 | Steuereingänge von 76,77 |
| F,F1,F2 | Führungsgrößen |
| I,I0,I1,I2,I3 | Ströme |
| P | Impulsfolge |
| t | Zeit |
| U1,U2,U3 | Spannungen |

**Patentansprüche**

1. Implantat zur elektrischen Impulsbeaufschlagung von lebendem Gewebe (11), mit einer steuerbaren Schalteinrichtung (6) und einer Ladekapazität (1), die an ihren beiden Seiten (4) und (8) zum Aufladen an eine Ladeschaltung (3) schaltbar ist und zum Entladen über die steuerbare Schalteinrichtung (6) mit mindestens zwei im Bereich des Gewebes (11) implantierten Elektroden (7,9) verbindbar ist, und mit einer Steueranordnung (12), die die von ihr gesteuerte Schalteinrichtung (6) mit einer vorgegebenen Schaltfrequenz ein- uns ausschaltet, **dadurch gekennzeichnet**, daß im Implantat zwischen der Schalteinrichtung (6) und den Verbindungen zu den Elektroden (7,9) Mittel (10,27,31) zum Glatten des elektrischen Stroms (I) durch die Elektroden (7,9) angeordnet sind und daß die Steueranordnung (12) Mittel (13,14,15,30,34) zum Ändern der Schaltfrequenz umfaßt, die die Schaltfrequenz wahrend der Dauer der Impulsbeaufschlagung des Gewebes (11) in der Weise ändern, daß der die Ladekapazität entladenden Strom (I) durch die verbindbaren Elektroden (7,9) einen vorgegebenen, von einem exponentiell abklingenden Stromverlauf abweichenden Verlauf aufweist.

2. Implantat nach Anspruch 1, **dadurch gekennzeichnet**, daß die Mittel zum Glätten des elektrischen Stroms (I) aus einer zwischen den Verbindungen zu den Elektroden (7,9) liegenden Glättungskapazität (10) bestehen.

3. Implantat nach Anspruch 2, **dadurch gekennzeichnet**, daß die Glattungskapazität (27) von den verbindbaren Elektrodenleitungen (25,26) gebildet wird, die die Verbindungen zu den Elektroden (7,9) mit der Schalteinrichtung (6) und der Ladekapazität (1) verbinden.

4. Implantat nach Anspruch 1, **dadurch gekennzeichnet**, daß die Mittel zum Glätten des elektrischen Stroms (I) aus einer Glattungsinduktivitat (31) bestehen, die in dem Stromweg von der Ladekapazität (1) und der Schalteinrichtung (6) zu den Verbindungen zu den Elektroden (7,9) angeordnet ist, und daß ein Stromventil (32) in Reihenschaltung mit der Glättungsinduktivität (31) zwischen den Verbindungen zu den Elektroden (7,9) angeordnet ist.

5. Implantat nach einem der vorangehenden Ansprüche, **dadurch gekennzeichnet**, daß die Mittel zum Ändern der Schaltfrequenz einen Impulsgeber (29) aufweisen, der eine vorgegebene Folge (P) von Einschalt-und Ausschaltimpulsen mit variablen Impulsdauern zum Ein-bzw. Ausschalten der steuerbaren Schalteinrichtung (6) erzeugt.

6. Implantat nach einem der Ansprüche 1 bis 4, **dadurch gekennzeichnet**, daß die Mittel zum Ändern der Schaltfrequenz eine Meßeinrichtung (13,30,34) zum Erfassen einer dem elektrischen Strom durch verbindbaren die Elektroden (7,9) oder der elektrischen Spannung an den verbindbaren Elektroden (7,9) entsprechenden Meßgröße aufweisen, daß eine Vorrichtung (14) zur Erzeugung einer dem vorgegebenen Stromverlauf entsprechenden Führungsgroße (F) vorgesehen ist und daß eine Vergleichseinrichtung (15) zum Vergleichen der Meßgröße mit der Führungsgröße (F) vorgesehen ist, wobei die Vergleichseinrichtung (15) für die steuerbare Schalteinrichtung (6) jedesmal dann ein Einschaltsignal erzeugt, wenn die Führungsgröße (F) die Meßgröße übersteigt, und ein Ausschaltsignal erzeugt, wenn die Führungsgroße (F) die Meßgröße unterschreitet.

7. Implantat nach einem der vorangehenden Ansprüche, **gekennzeichnet durch** eine Impedanzmeßeinrichtung (69) zur Erfassung der elektrischen Impedanz zwischen den Verbindungen zu den Elektroden (47,48,50) wahrend vorgegebener Meßzeiten und zur Steuerung der Mittel (54,56,58,60,62,64) zur Anderung der Schaltfrequenz in Abhangigkeit von der gemessenen Impedanz.

8. Implantat nach einem der vorangehenden Ansprüche, **dadurch gekennzeichnet**, daß die Mittel (13,14,15) zum Ändern der Schaltfrequenz mit einem Parameterspeicher (22) verbunden sind, in dem Parameterwerte für die Änderung der Schaltfrequenz abgespeichert sind, und daß der Parameterspeicher (22) mit einer Telemetrieeinrichtung (23) zur Übertragung der Parameterwerte zwischen dieser und einem Programmiergerat (24) verbunden ist.

9. Implantat nach einem der vorangehenden Ansprüche, **dadurch gekennzeichnet**, daß die Ladekapazitat (74) oder zumindest eine weitere, separat aufladbare Ladekapazitat (39) an ihren beiden Seiten (41,42) über eine weitere steuerbare Schalteinrichtung (44) mit den Verbindungen zu einer der verbindbaren Elektroden (50) und den Verbindungen zu einer weiteren verbindbaren Elektrode (48) verbunden ist, daß zwischen der weiteren Schalteinrichtung und den Verbindungen zu der verbindbaren Elektrode (50) und den Verbindungen zu der weiteren verbindbaren Elektrode (48) Mittel (53) zum Glätten des elektrischen Stroms durch die weitere verbindbaren Elektrode (48) angeordnet sind und daß eine weitere Steueranordnung (60,62,64) zum Einund Ausschalten der weiteren steuerbaren Schalteinrichtung (44) mit einer änderbaren Schaltfrequenz vorgesehen ist.

## Claims

1. Implant for applying electrical pulses to living tissue (11), having a controllable switching device (6) and a charging capacitor (1) which can be switched to a charging circuit arrangement (3) on its two sides (4) and (8) for charging purposes and can be connected to at least two electrodes (7, 9), which are implanted in the region of the tissue (11), by way of the controllable switching device (6), for discharging purposes, and having a control arrangement (12) which switches the switching device (6), which is controlled by it, on and off with a given switching frequency, characterised in that means (10, 27, 31) for smoothing the electric current (I) through the electrodes (7, 9) are arranged in the implant between the switching device (6) and the connections to the electrodes (7, 9), and in that the control arrangement (12) comprises means (13, 14, 15, 30, 34) for changing the switching frequency that change the switching frequency during the length of time during which pulses are applied to the tissue (11) in such a way that the current (I), which discharges the charging capacitor, through the connectable electrodes (7, 9) has a given characteristic that deviates from an exponentially decaying current characteristic.

2. Implant according to claim 1, characterised in that the means for smoothing the electric current (I) consist of a smoothing capacitor (10) which lies between the connections to the electrodes (7, 9).

3. Implant according to claim 2, characterised in that the smoothing capacitor (27) is formed by the connectable electrode lines (25, 26) which connect the connections to the electrodes (7, 9) to the switching device (6) and the charging capacitor (1).

4. Implant according to claim 1, characterised in that the means for smoothing the electric current (I) consist of a smoothing inductor (31) which is arranged in the current path from the charging capacitor (1) and the switching device (6) to the connections to the electrodes (7, 9), and in that a flow valve (32) is arranged in a series circuit arrangement with the smoothing inductor (31) between the connections to the electrodes (7, 9).

5. Implant according to one of the preceding claims, characterised in that the means for changing the switching frequency have a pulse generator (29) which generates a given sequence (P) of on- and off-pulses with variable pulse durations for switching on and off the controllable switching device (6).

6. Implant according to one of claims 1 to 4, characterised in that the means for changing the switching frequency have a measuring device (13, 30, 34) for measuring a measured variable that corresponds to the electric current through the connectable electrodes (7, 9) or the electric voltage at the connectable electrodes (7, 9), in that an arrangement (14) for generating a reference variable (F) which corresponds to the given current characteristic is provided, and in that a comparison device (15) for comparing the measured variable with the reference variable (F) is provided, wherein the comparison device (15) generates an on-signal for the controllable switching device (6) each time the reference variable (F) exceeds the measured variable and generates an off-signal if the reference variable (F) falls short of the measured variable.

7. Implant according to one of the preceding claims, characterised by an impedance-measuring device (69) for measuring the electrical impedance between the connections to the electrodes (47, 48, 50) during given measuring times and for controlling the means (54, 56, 58, 60, 62, 64) for changing the switching frequency as a function of the measured impedance.

8. Implant according to one of the preceding claims, characterised in that the means (13, 14, 15) for changing the switching frequency are connected to a parameter memory (22) in which parameter values for changing the switching frequency are stored, and in that the parameter memory (22) is connected to a telemetry device (23) for transmitting the parameter values between the latter and a programming unit (24).

9. Implant according to one of the preceding claims, characterised in that the charging capacitor (74) or at least one further separately chargeable charging capacitor (39) is connected on its two sides (41, 42) by way of a further controllable switching device (44) to the connections to one of the connectable electrodes (50) and the connections to a further connectable electrode (48), in that means (53) for smoothing the electric current through the further connectable electrode (48) are arranged between the further switching device and the connections to the connectable electrode (50) and the connections to the further connectable electrode (48), and in that a further control arrangement (60, 62, 64) is provided for switching the further controllable switching device (44) on and off with a variable switching frequency.

## Revendications

1. Implant pour appliquer des impulsions électriques à un tissu vivant (11), comportant un dispositif de commutation (6) qui peut être commandé et une capacité de charge (1) qui peut être raccordée par ses deux côtés (4) et (8) pour la charge à un circuit de charge (3) et qui peut être reliée pour la décharge par l'intermédiaire du dispositif de commutation (6) pouvant être commandé à au moins deux électrodes (7, 9) implantées dans la zone du tissu (11), et un dispositif de commande (12) qui branche et débranche avec une fréquence de commutation prescrite le dispositif de commutation (6) qu'il commande, caractérisé par le fait qu'il est agencé dans l'implant, entre le dispositif de commutation (6) et les liaisons vers les électrodes (7, 9), des moyens (10, 27, 31) pour lisser le courant électrique (I) passant dans les électrodes (7, 9) et le dispositif de commande (12) comprend des moyens (13, 14, 15, 30, 34) pour modifier la fréquence de commutation, lesquels moyens modifient la fréquence de commutation pendant la durée de l'application d'impulsions au tissu (11) de telle sorte que le courant (I) déchargeant la capacité de charge dans les électrodes (7, 9) pouvant être reliées a une allure prescrite s'écartant d'une allure de courant décroissante de façon exponentielle.

2. Implant selon la revendication 1, caractérisé par le fait que les moyens pour lisser le courant électrique (I) consistent en une capacité de lissage (10) placée entre les liaisons vers les électrodes (7, 9).

3. Implant selon la revendication 2, caractérisé par le fait que la capacité de lissage (27) est formée par les lignes d'électrodes (25, 26), pouvant être reliées, qui relient les liaisons vers les électrodes (7, 9) au dispositif de commutation (6) et à la capacité de charge (1).

4. Implant selon la revendication 1, caractérisé par le fait que les moyens pour lisser le courant électrique (I) consistent en une inductance de lissage (31), qui est agencée dans le trajet du courant de la capacité de charge (1) et du dispositif de commutation (6) aux liaisons vers les électrodes (7, 9), et qu'une valve de courant (32) est agencée en série avec l'inductance de lissage (31) entre les liaisons vers les électrodes (7, 9).

5. Implant selon l'une des revendications précédentes, caractérisé par le fait que les moyens pour modifier la fréquence de commutation comportent un générateur d'impulsions (29) qui produit une suite prescrite (P) d'impulsions de branchement et de débranchement à durées d'impulsions variables en vue du branchement et du débranchement du dispositif de commutation (6) pouvant être commandé.

6. Implant selon l'une des revendications 1 à 4, caractérisé par le fait que les moyens pour modifier la fréquence de commutation comportent un dispositif de mesure (13, 30, 34) pour détecter une grandeur de mesure correspondant au courant électrique dans les électrodes (7, 9) pouvant être reliées ou correspondant à la tension électrique aux électrodes (7, 9) pouvant être reliées, qu'un dispositif (14) pour produire une grandeur de commande (F) correspondant à l'allure de courant prescrite est prévu et qu'un dispositif de comparaison (15) pour comparer la grandeur de mesure à la grandeur de commande (F) est prévu, le dispositif de comparaison (15) produisant pour le dispositif de commutation (6) pouvant être commandé un signal de branchement chaque fois que la grandeur de commande (F) dépasse la grandeur de mesure et un signal de débranchement chaque fois que la grandeur de commande (F) passe sous la grandeur de mesure.

7. Implant selon l'une des revendications précédentes, caractérisé par un dispositif de mesure d'impédance (69) pour détecter l'impédance électrique entre les liaisons vers les électrodes (47, 48, 50) pendant des temps de mesure prescrits et pour commander les moyens (54, 56, 58, 60, 62, 64) destinés à modifier la fréquence de commutation en fonction de l'impédance mesurée.

8. Implant selon l'une des revendications précédentes, caractérisé par le fait que les moyens (13, 14, 15) pour modifier la fréquence de commutation sont reliés à une mémoire de paramètres (22), dans laquelle des valeurs de paramètres sont mémorisées pour la modification de la fréquence de commutation, et que la mémoire de paramètres (22) est reliée à un dispositif de télémétrie (23) pour transmettre les valeurs de paramètres entre celui-ci et un appareil de programmation (24).

9. Implant selon l'une des revendications précédentes, caractérisé par le fait que la capacité de charge (74) ou au moins une autre capacité de charge (39), pouvant être chargée séparément, est reliée par ses deux côtés (41, 42) par l'intermédiaire d'un autre dispositif de commutation (44) pouvant être commandé aux liaisons vers une des électrodes (50) pouvant être reliées et aux liaisons vers une autre électrode (48) pouvant être reliée, qu'il est agencé entre l'autre dispositif de commutation et les liaisons vers l'électrode (50) pouvant être reliée et les liaisons vers l'autre électrode (48) pouvant être reliée, des moyens (53) pour lisser le courant électrique passant dans l'autre électrode (48) pouvant être reliée et qu'il est prévu un autre dispositif de commande (60, 62, 64) pour brancher et débrancher avec une fréquence de commutation modifiable l'autre dispositif de commutation (44) pouvant être commandé.

FIG.1

FIG. 2

FIG.3

FIG. 4

FIG. 5

FIG.6

FIG.7

FIG. 8

FIG. 9

FIG. 10